# EUROPÄISCHE PATENTANMELDUNG

(11) **EP 2 272 425 A2**
(43) Veröffentlichungstag der Anmeldung: **12.01.2011**
(21) Anmeldenummer: 10185806.6
(22) Anmeldetag: 07.10.2004
(51) Int. Cl.: A61B 5/00, A61B 19/00

(54) **Erzeugen eines mit Vermerken versehenen Bildes in sterilem Arbeitsbereich**

(30) Priorität: 17.10.2003 DE 10349649
(62) Teilanmeldung aus: 04790168.1
(71) Anmelder: Karl Storz GmbH & Co. KG, 78532 Tuttlingen (DE)
(72) Erfinder: Nölle, Martin, 78315 Radolfzell (DE); Hilzinger, Hans-Uwe, 78532 Tuttlingen (DE); Stillor, Heinz-Werner, 79798 Jestetten (DE)
(74) Vertreter: Witte, Weller & Partner

(57) **Zusammenfassung**

Eine Vorrichtung zum Erzeugen eines mit Bearbeitungsvermerken versehenen Bildes besitzt eine in einem sterilen Arbeitsbereich (56) angeordnete medizinische Anzeigeeinheit (12) zum Darstellen eines primären Bildes. Des Weiteren ist ein Eingabegerät zum Erzeugen von Eingabedaten (24) in dem sterilen Arbeitsbereich (56) vorhanden, wobei das Eingabegerät vorzugsweise ein berührungsempfindlicher Bildschirm (12) ist. Außerhalb des sterilen Bereichs (56) ist eine Bildverarbeitungseinheit (52) angeordnet, die dazu ausgebildet ist, Eingabedaten (24) und das primäre Bild zu einem sekundären Bild (72) zusammenzuführen. Das sekundäre Bild (72) wird anschließend über eine Schnittstelle (18, 32) wieder an die im sterilen Bereich (56) angeordnete Anzeigeeinheit (12) übertragen.

## Beschreibung

Die vorliegende Erfindung betrifft eine Vorrichtung zum Erzeugen eines mit Bearbeitungsvermerken versehenen Bildes in einem sterilen Arbeitsbereich einer medizinischen Einrichtung.

In der modernen Medizin werden Behandlungen mehr und mehr unter Verwendung von technischen bildgebenden Verfahren durchgeführt. Dabei werden beispielsweise miniaturisierte Kameras in den Körper eines Patienten eingeführt, und das mit der Kamera aufgenommene Bild wird dem behandelnden Arzt auf einem in seinem Arbeitsbereich installierten Monitor angezeigt. Der behandelnde Arzt kann auf diese Weise beispielsweise ein inneres Organ oder ein Gelenk zu Diagnosezwecken begutachten und auch chirurgische Eingriffe minimal-invasiv durchführen. Um die Behandlung eines Patienten so effizient und einfach wie möglich zu machen, ist es dabei von Vorteil, wenn der Monitor im Arbeitsbereich des Arztes, d.h. im sterilen Bereich, angeordnet ist. Der Arzt hat damit die Möglichkeit, sämtliche Eingriffe, die er am Patienten vornimmt, "live" auf dem Monitor zu verfolgen, wobei das entsprechende Monitorbild beispielsweise mit einer endoskopischen Kamera aufgenommen wird. Die Erfindung ist allerdings nicht auf Anwendungen beschränkt, bei denen Bilder mit Hilfe von endoskopischen Kameras erzeugt werden. Wenngleich es sich dabei um einen bevorzugten Anwendungsfall handelt, spielt die Herkunft der Bilder für die vorliegende Erfindung eine untergeordnete Rolle. Beispielsweise können die Bilder auch Röntgenbilder oder Aufnahmen aus einem Archiv sein, die beispielsweise zu Vergleichszwecken auf den Monitor im Arbeitsbereich des Arztes eingespielt werden.

Darüber hinaus müssen der oder die behandelnden Ärzte bei einem chirurgischen Eingriff heutzutage eine Vielzahl von Geräten bedienen, die häufig ebenfalls im sterilen Bereich angeordnet sind. Um die zahlreichen Geräte möglichst effizient und komfortabel bedienen zu können, kommen moderne Netzwerk- und Kommunikationstechnologien zum Einsatz. Beispielsweise bietet die Anmelderin der vorliegenden Erfindung unter der Bezeichnung "OR-1" ein System für Operationssäle an, bei dem verschiedene Komponenten innerhalb und außerhalb des sterilen Arbeitsbereichs miteinander vernetzt sind. Aus US 5,788,688 ist des Weiteren ein integriertes Bedienungs- und Steuerungssystem für chirurgische Eingriffe bei einem Patienten bekannt, bei dem von solchen Möglichkeiten einschließlich der Nutzung bildgebender Verfahren Gebrauch gemacht wird.

Um die Einsatzmöglichkeiten eines solchen Systems weiter zu verbessern, ist es wünschenswert, das angezeigte Bild mit Kommentaren, Anmerkungen, Markierungen und dergleichen, allgemein also mit Bearbeitungsvermerken, zu versehen. Hierdurch wird beispielsweise eine Diskussion zwischen mehreren behandelnden Ärzten bei der Erstellung eines Befundes erleichtert. Auch zu Ausbildungs- und Schulungszwecken ist es wünschenswert, wenn beispielsweise ein erfahrener Arzt Bereiche im angezeigten Bild markieren und mit Kommentaren versehen kann.

Für Anwendungen außerhalb des medizinischen Bereichs, beispielsweise für Büroanwendungen und andere kommerzielle Zwecke, gibt es Geräte, die es erlauben, ein auf einer Anzeigeeinheit (Monitor, Beamer oder ähnliches) angezeigtes Bild mit Kommentaren und Markierungen zu versehen. Beispielsweise bietet die Firma Boeckeler Instruments, Inc. mit Sitz in Tucson, Arizona, USA entsprechende Geräte unter der Bezeichnung "Pointmaker®" an. Diese Geräte haben jedoch den Nachteil, dass sie nicht für Anwendungen im medizinischen Bereich konzipiert sind und daher nicht im sterilen Arbeitsbereich eingesetzt werden dürfen.

Eine Möglichkeit, um dem oben beschriebenen Bedürfnis Rechnung zu tragen, wäre die Nutzung der kommerziell erhältlichen Geräte außerhalb des sterilen Arbeitsbereichs, wobei das betreffende Bild aus dem sterilen Arbeitsbereich dann zusätzlich noch einmal auf einen zweiten, außerhalb des sterilen Bereichs angeordneten Monitor übertragen werden müsste. Nachteilig ist hierbei, dass der behandelnde Arzt den sterilen Arbeitsbereich verlassen muss, um Bearbeitungsvermerke in das angezeigte Bild einzufügen.

Eine weitere Möglichkeit wäre, kommerziell erhältliche Geräte so umzurüsten, dass sie die Anforderungen für eine Verwendung im sterilen Arbeitsbereich eines Operationssaals erfüllen. Alternativ hierzu könnte man darangehen, ein für medizinische Anwendungen geeignetes Gerät zu entwickeln, das die gewünschte Funktionalität, wie sie von kommerziell erhältlichen Geräten bekannt ist, von sich aus beinhaltet. Beide Ansätze sind jedoch aufwendig und damit teuer.

Vor diesem Hintergrund ist es eine Aufgabe der vorliegenden Erfindung, auf einfache und kostengünstige Weise eine Möglichkeit bereitzustellen, die es einem behandelnden Arzt erlaubt, ein medizinisches Bild, insbesondere ein endoskopisches Videobild, vom sterilen Bereich aus mit Bearbeitungsvermerken zu versehen.

Die Aufgabe wird gemäß einem Aspekt der Erfindung durch eine Vorrichtung der eingangs genannten Art gelöst, mit
- einer in dem sterilen Arbeitsbereich angeordneten medizinischen Anzeigeeinheit zum Darstellen eines primären Bildes,
- einem in dem sterilen Arbeitsbereich angeordneten Eingabegerät zum Erzeugen von Eingabedaten, wobei die Eingabedaten den Bearbeitungsvermerken entsprechen, und
- einer außerhalb des sterilen Bereichs angeordneten Bildverarbeitungseinheit, die dazu ausgebildet ist, die Eingabedaten und das primäre Bild aufzunehmen und zu einem sekundären Bild zusammenzuführen,
- wobei die medizinische Anzeigeeinheit und die Bildverarbeitungseinheit über eine Schnittstelle verbunden sind, so dass das sekundäre Bild an die Anzeigeeinheit übertragbar und dort darstellbar ist.

Die Anmeldung offenbart außerdem ein Verfahren zum Erzeugen eines mit Bearbeitungsvermerken versehenen Bildes in einem sterilen Arbeitsbereich einer medizinischen Einrichtung, wobei das Verfahren folgende Schritte aufweist:
- Darstellen eines primären Bildes auf einer medizinischen Anzeigeeinheit, die in dem sterilen Arbeitsbereich angeordnet ist,
- Bereitstellen eines Eingabegerätes zum Erzeugen von Eingabedaten in dem sterilen Arbeitsbereich, wobei die Eingabedaten den Bearbeitungsvermerken entsprechen,
- Einlesen der Eingabedaten und Übertragen der Eingabedaten an eine Bildverarbeitungseinheit, die außerhalb des sterilen Bereichs angeordnet ist,
- Übertragen des primären Bildes an die Bildverarbeitungseinheit,
- Erzeugen eines sekundären Bildes in der Bildverarbeitungseinheit durch Zusammenführen des primären Bildes und der Eingabedaten, und
- Übertragen des sekundären Bildes an die Anzeigeeinheit und Darstellen des sekundären Bildes auf der Anzeigeeinheit.

Das vorgeschlagene Verfahren und die entsprechende Vorrichtung beruhen damit auf der Idee, sowohl das primäre, mit den Bearbeitungsvermerken zu versehende Bild als auch die Eingabedaten, die die Bearbeitungsvermerke repräsentieren, aus dem sterilen Bereich "nach außen", d.h. in einen nicht-sterilen Bereich, zu übertragen. Dort können das primäre Bild und die Eingabedaten dann auf relativ einfache und kostengünstige Weise zusammengeführt werden, wobei vorteilhafterweise für diesen Teilschritt eine kommerziell erhältliche und damit für medizinische Anwendungen an sich nicht zugelassene Bildverarbeitungseinheit verwendet wird. Statt eine kommerziell erhältliche Bildverarbeitungseinheit für eine Anwendung in medizinischen Bereichen aufzurüsten, wird hiernach also die Aufbereitung des mit Bearbeitungsvermerken versehenen Bildes in einem unkritischen Bereich ausgelagert. Die "Auslagerung" erfolgt jedoch so, dass ein behandelnder Arzt die Eingabedaten trotzdem von seinem sterilen Arbeitsbereich aus erzeugen kann. Der vorgeschlagene Weg verbindet somit die Vorteile der oben genannten Alternativlösungen.

Nachteil der vorgeschlagenen Vorgehensweise ist, dass sowohl die Eingabedaten als auch das primäre Bild zu der entfernt angeordneten Bildverarbeitungseinheit übertragen werden müssen, was einen nicht unerheblichen Kommunikationsaufwand bedeutet. Es hat sich jedoch gezeigt, dass dieser Aufwand einfacher und kostengünstiger zu bewältigen ist, als man zunächst vermuten konnte. Die genannte Aufgabe ist daher vollständig gelöst.

Darüber hinaus besitzen das neue Verfahren und die neue Vorrichtung den Vorteil, dass das mit den Bearbeitungsvermerken versehene, sekundäre Bild, für eine weitere Verarbeitung außerhalb des sterilen Bereichs unmittelbar zur Verfügung steht. Diese weitere Verarbeitung kann beispielsweise die Anzeige auf einem abgesetzten Schulungsmonitor und/oder die Archivierung in einer elektronischen Patientenkartei sein. Das neue System bietet daher einen erweiterten Anwendungsbereich.

In einer Ausgestaltung der Erfindung ist das primäre Bild eine medizinische Aufnahme eines Patienten, insbesondere eine in dem sterilen Arbeitsbereich endoskopisch erzeugte Aufnahme.

In dieser bevorzugten Ausgestaltung kommen die Vorteile der Erfindung besonders deutlich zur Geltung. So ist die Analyse einer medizinischen Aufnahme eines Patienten und insbesondere die Analyse einer aktuellen Aufnahme des Patienten während einer Behandlung ein Anwendungsfall, der durch die Bereitstellung von Bearbeitungsvermerken einfacher und übersichtlicher wird. Beispielsweise können Missverständnisse bei einer schwierigen Operation, an der mehrere behandelnde Ärzte beteiligt sind, in dieser bevorzugten Ausgestaltung einfacher vermieden werden.

In einer weiteren Ausgestaltung ist das Eingabegerät ein berührungsempfindlicher Bildschirm, der vorzugsweise auch die Anzeigeeinheit bildet.

Berührungsempfindliche Bildschirme (so genannte "touch-screens") sind an sich bekannt und werden auch bereits als Anzeige- und Bedieneinheiten in medizinischen Einrichtungen eingesetzt. In der bevorzugten Ausgestaltung besitzt ein berührungsempfindlicher Bildschirm den besonderen Vorteil, dass der behandelnde Arzt Bearbeitungsvermerke sehr einfach und schnell allein mit Hilfe seiner Hände erzeugen kann. Darüber hinaus ist ein berührungsempfindlicher Bildschirm hervorragend als Eingabegerät für den sterilen Arbeitsbereich geeignet, und er benötigt insbesondere in der bevorzugten Ausgestaltung keinen zusätzlichen Platz. Die Eingabedaten sind in der bevorzugten Ausgestaltung insbesondere die Koordinaten auf dem berührungsempfindlichen Bildschirm, an denen eine Berührung durch den behandelnden Arzt detektiert wird.

In einer weiteren Ausgestaltung werden die Eingabedaten über eine galvanische Trennstelle an die Bildverarbeitungseinheit übertragen.

Diese Ausgestaltung besitzt den Vorteil, dass der sterile Arbeitsbereich und die darin verwendeten Geräte sehr zuverlässig von dem nicht-sterilen Außenbereich abgeschottet sind. Die Funktionssicherheit und Funktionszuverlässigkeit der Gesamteinrichtung wird hierdurch erhöht.

In einer weiteren Ausgestaltung werden die Eingabedaten bei der Übertragung an die Bildverarbeitungseinheit einer Protokollumsetzung unterzogen. Dementsprechend ist in einer bevorzugten Ausgestaltung der neuen Vorrichtung ein Protokollumsetzer vorhanden, der weiter bevorzugt in Form eines Schnittstellenmoduls, insbesondere einer Schnittstellenkarte, ausgebildet ist.

Diese Ausgestaltung besitzt den Vorteil, dass allein durch Adaptierung des Protokollumsetzers eine beliebige und gegebenenfalls austauschbare Bildverarbeitungseinheit verwendet werden kann. Insbesondere wird hierdurch die Verwendung von kommerziell erhältlichen Bildverarbeitungseinheiten erleichtert, was eine besonders kostengünstige Realisierung ermöglicht.

In einer weiteren Ausgestaltung werden die Eingabedaten in einem digitalen Format an die Bildverarbeitungseinheit übertragen. Des weiteren ist es bevorzugt, wenn das primäre Bild als analoges Bildsignal an die Bildverarbeitungseinheit übertragen wird.

Grundsätzlich könnten die Eingabedaten auch in analoger Form bzw. das primäre Bild in digitaler Form an die Bildverarbeitungseinheit übertragen werden. Die bevorzugten Ausgestaltungen erleichtern demgegenüber jedoch die praktische Realisierung. So kann insbesondere eine Protokollumsetzung bei digitalen Eingabedaten relativ einfach und kostengünstig per Software realisiert werden. Die Übertragung des primären Bildes als analoges Bildsignal ist demgegenüber mit heutigen Mitteln einfacher und schneller als in digitaler Form zu realisieren. Außerdem kann in diesem Fall auf kostengünstige und erprobte Komponenten zurückgegriffen werden.

In einer weiteren Ausgestaltung wird das sekundäre Bild auf einer zweiten Anzeigeeinheit dargestellt, die außerhalb des sterilen Arbeitsbereichs angeordnet ist.

Diese Ausgestaltung nutzt die Möglichkeiten des neuen Verfahrens in besonders vorteilhafter Weise, da hierdurch beispielsweise Ausbildung und Schulungen, Datenarchivierung und auch Videokonferenzen, an denen weit entfernt sitzende Fachleute beteiligt sind, wesentlich vereinfacht werden.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

In der einzigen Figur ist ein bevorzugtes Ausführungsbeispiel der Erfindung in einem vereinfachten Blockschaltbild dargestellt.

In der Figur ist eine medizinische Einrichtung in ihrer Gesamtheit mit der Bezugsziffer 10 bezeichnet.

Die Einrichtung 10 beinhaltet eine Anzeigeeinheit 12, die in dem bevorzugten Ausführungsbeispiel ein berührungsempfindlicher Bildschirm ist. Alternativ hierzu könnte die Anzeigeeinheit 12 jedoch auch ein analoger Röhrenmonitor, ein einfacher LCD-Monitor, ein Beamer oder eine beliebige andere Anzeigeeinheit sein. In dem bevorzugten Ausführungsbeispiel bildet der berührungsempfindliche Bildschirm 12 gleichzeitig auch ein Eingabegerät, was hier durch die Hand 14 eines Bedieners dargestellt ist.

Mit der Bezugsziffer 16 ist ein für medizinische Zwecke ausgebildeter PC (personal computer) bezeichnet, der mit einer so genannten Framegrabberkarte 18 ausgerüstet ist. Aufgabe der Framegrabberkarte 18 ist es, ein analoges Videosignal, insbesondere ein so genanntes S-VHS-Signal aufzunehmen, zu digitalisieren und anschließend der internen Verarbeitungseinheit (hier nicht dargestellt) des PCs zur Verfügung zu stellen.

Der PC 16 ist über zwei Verbindungen 20, 22 mit dem berührungsempfindlichen Bildschirm 12 verbunden. Die Verbindung 20 ist eine übliche Videosignalverbindung, beispielsweise eine VGA-Verbindung oder eine DVI-Verbindung, über die die Anzeigeeinheit an einen PC angeschlossen wird. Der PC 16 steuert die Anzeigeeinheit 12 folglich über die Verbindung 20 an.

Die Verbindung 22 ist in dem bevorzugten Ausführungsbeispiel eine digitale Datenverbindung, über die Eingabedaten 24 von dem berührungsempfindlichen Bildschirm 12 an den PC 16 übertragen werden. Die Verbindung 22 ist hier insbesondere eine serielle RS-232-Verbindung. Es könnte jedoch beispielsweise auch eine PS2-verbindung, eine USB-Verbindung oder eine parallele Datenverbindung sein. Der PC 16 wertet die Eingabedaten 24 aus, als wären es Eingabedaten von einer Maus.

Mit der Bezugsziffer 26 ist eine Kameraeinheit bezeichnet, die hier insbesondere eine endoskopische Kamera und ein entsprechendes Steuergerät (nicht gesondert dargestellt) beinhaltet. Mit der Kameraeinheit 26 wird beispielsweise ein inneres Organ 28 eines hier im Übrigen nicht dargestellten Patienten aufgenommen. Die Kameraeinheit 26 ist über einen Trennverstärker 30 oder einen Trenntrafo mit einer sogenannten Videokreuzschiene (video crossbar) verbunden. Der Trennverstärker 30 kann abweichend von der hier gezeigten Blockschaltbilddarstellung beispielsweise auch in der Kameraeinheit 26 bzw. der erwähnten Steuereinheit integriert sein.

Die Videokreuzschiene 32 besitzt eine Vielzahl von Eingängen 34, 36, 38, 40, an die jeweils eine Videosignalquelle angeschlossen werden kann. Des Weiteren besitzt die Videokreuzschiene 32 eine Reihe von Ausgängen 42, 44, 46, an denen Videosignale ausgegeben werden. In dem gezeigten Ausführungsbeispiel sind sowohl die Signale an den Videoeingängen 34-40 als auch die Videosignale an den Ausgängen 42-46 S-VHS-Signale. Es können hier jedoch auch andere Arten von Videosignalen verwendet werden.

Die Kameraeinheit 26 ist über den Trennverstärker 30 an den Eingang 34 der Videokreuzschiene 32 angeschlossen. An den Eingang 36 ist über einen weiteren Trennverstärker eine weitere Kameraeinheit 48 angeschlossen. Die Kameraeinheit 48 ist hier beispielsweise im Unterschied zur Kameraeinheit 26 eine 3D-Kamera mit entsprechender Steuereinheit, die es ermöglicht, dreidimensionale Bilder aufzunehmen. Des Weiteren können hier auch andere Arten von Videosignalquellen angeschlossen sein.

Mit der Bezugsziffer 50 ist hier beispielhaft für eine andere Videoquelle ein CD- oder DVD-Player bezeichnet, der an den Eingang 38 der Videokreuzschiene 32 angeschlossen ist. Der CD- oder DVD-Player 50 dient beispielsweise dazu, archiviertes Bildmaterial zur Ausgabe auf der Anzeigeeinheit 12 bereitzustellen.

An den Eingang 40 der Videokreuzschiene 32 ist eine Bildverarbeitungseinheit 52 angeschlossen. Die Bildverarbeitungseinheit 52 ist bevorzugt eine kommerziell erhältliche Bildverarbeitungseinheit zum Überlagern von handschriftlichen Bearbeitungsvermerken auf einem Videobild. Bevorzugt handelt es sich hier um eine Einheit, die von der Firma Boeckeler Instruments Inc., 4650 South Butterfield Drive, Tucson, Arizona, USA unter der Bezeichnung "Pointmaker®" PVI-44 angeboten wird.

Die Bildverarbeitungseinheit 52 ist ferner auch an den Ausgang 46 der Videokreuzschiene 32 angeschlossen, so dass eine Schleife gebildet wird. An den Ausgang 44 der Videokreuzschiene 32 ist hier eine zweite Anzeigeeinheit 54 angeschlossen. Der Ausgang 42 ist über einen weiteren Trennverstärker 30 mit der Framegrabberkarte 18 verbunden. Es versteht sich, dass die gezeigte Konfiguration beispielhaft ist und insbesondere die zweite Kameraeinheit 48, der CD-/DVD-Player 50 und die zweite Anzeigeeinheit 54 für die Realisierung der Erfindung nicht erforderlich sind. Darüber hinaus ist auch die Verwendung der Videokreuzschiene 32 hier nur ein bevorzugtes Ausführungsbeispiel. Aufgabe der Videokreuzschiene 32 ist es nämlich, die an den verschiedenen Eingängen 34-40 anliegenden Videosignale wahlweise an einen oder mehrere der Ausgänge 42-46 durchzuschalten. Wird nur eine Videoquelle, beispielsweise also nur die Kameraeinheit 26, verwendet, genügt es, deren Videosignal über einen Splitter (hier nicht dargestellt) der Framegrabberkarte 18 und der Bildverarbeitungseinheit 52 zuzuführen. Bevorzugt werden jedoch auch in diesem Fall die Trennverstärker 30 verwendet, um eine galvanische Trennung zwischen dem sterilen Arbeitsbereich (hier mit Bezugsziffer 56 bezeichnet) und dem nicht-sterilen Bereich 58 zu gewährleisten.

Die Trennung zwischen dem sterilen Bereich 56 und dem nicht-sterilen Bereich 58 ist hier durch eine Linie 60 symbolisch angedeutet.

Mit der Bezugsziffer 62 ist hier des weiteren ein Digitizertablett bezeichnet, auf dem man mit Hilfe eines Stiftes 64 manuelle Eingaben machen kann, die von der Bildverarbeitungseinheit 52 eingelesen und verarbeitet werden. Dementsprechend ist das Digitizertablett 62 hier über einen entsprechenden Anschluss mit der Bildverarbeitungseinheit 52 verbunden. Auch das Digitizertablett 62 ist für die praktische Realisierung der Erfindung nicht zwingend erforderlich und daher optional.

Mit der Bezugsziffer 66 ist des Weiteren eine Schnittstelleneinheit bezeichnet, die im hiesigen Ausführungsbeispiel mit einem Schnittstellenmodul 68 in Form einer Einsteckkarte (Platine) ausgerüstet ist. Ausgangsseitig ist die Schnittstelleneinheit 66 über das Schnittstellenmodul 68 mit einem weiteren Eingang der Bildverarbeitungseinheit 52 verbunden. Eingangsseitig ist die Schnittstelleneinheit 66 über einen weiteren Trennverstärker 30 (der auch in der Schnittstelleneinheit 66 integriert sein kann) mit dem PC 16 verbunden. In einem bevorzugten Ausführungsbeispiel ist die Verbindung zwischen dem PC 16 und der Schnittstelleneinheit 66 eine digitale Busverbindung, insbesondere eine CAN-basierte Busverbindung, wie sie von der Anmelderin der vorliegenden Erfindung unter der Bezeichnung SCB speziell für die Vernetzung von medizinischen Geräten angeboten wird. Die Verbindung zwischen dem Schnittstellenmodul 68 und der Bildverarbeitungseinheit 52 ist in dem bevorzugten Ausführungsbeispiel eine RS-232-Verbindung. Alternativ könnte es sich hier jedoch auch um eine USB-Verbindung, eine parallele Datenverbindung oder eine andere, bevorzugt standardisierte Datenverbindung handeln. Das Schnittstellenmodul 68 führt eine Protokollumsetzung durch, indem es von dem PC 16 empfangene Eingabedaten 24 (in dem bevorzugten Ausführungsbeispiel im Datenformat der SCB-Verbindung) in das digitale Datenformat der RS-232-Verbindung zur Bildverarbeitungseinheit 52 umsetzt.

Die Funktionsweise der Einrichtung 10 ist nun wie folgt:
Mit der Kameraeinheit 26 wird das Organ 28 des Patienten aufgenommen. Das Videosignal der Kameraeinheit 26 wird über die Videokreuzschiene 32 der Framegrabberkarte 18 zugeführt, von dieser digitalisiert und anschließend über den PC 16 auf dem berührungsempfindlichen Bildschirm 12 ausgegeben. Dieses ausgegebene Bild ist das primäre Bild im Sinne der vorliegenden Erfindung.

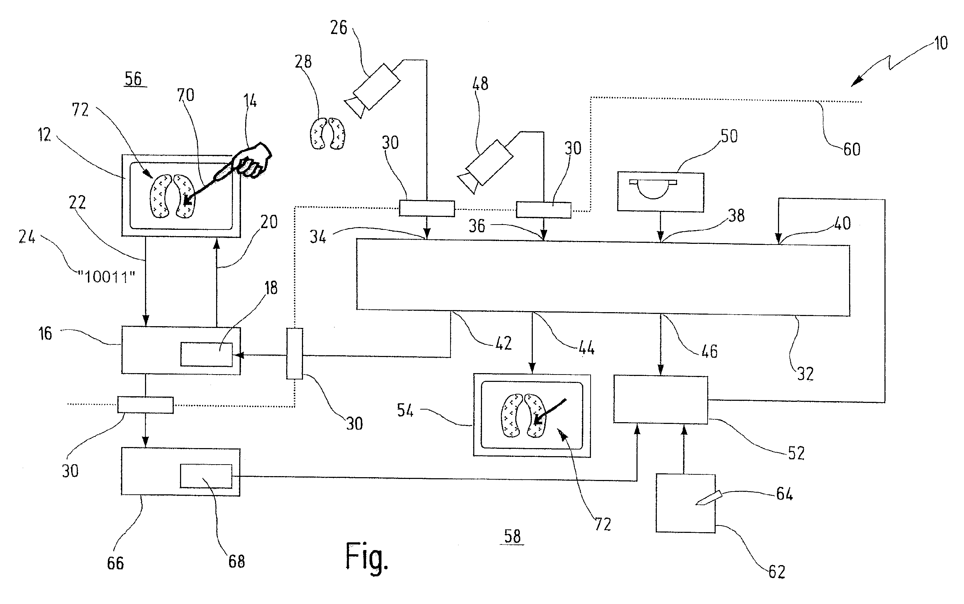

Der behandelnde Arzt hat nun die Möglichkeit, mit seiner Hand 14 Bereiche auf dem berührungsempfindlichen Bildschirm zu markieren. Die entsprechenden Koordinaten werden als Eingabedaten 24 an den PC 16 übertragen und dort als Mausbewegungen detektiert. Durch die Eingabedaten kann der behandelnde Arzt u.a. an den PC 16 angeschlossene medizinische Geräte (hier nicht dargestellt), wie beispielsweise Insufflatoren, einen Operationstisch oder auch die OP-Beleuchtung, steuern. Des Weiteren kann er den PC 16 durch einen entsprechenden Eingabebefehl in einen "Markierungs- oder Zeichenmodus" versetzen, in dem nachfolgende Eingaben auf dem berührungsempfindlichen Bildschirm 12 als Bearbeitungsvermerke interpretiert werden. Der PC 16 setzt die dann detektierten Mausbewegungen in graphische Symbole um. Beispielhaft ist in der Figur ein graphisches Symbol in Form eines von Hand gezeichneten Markierungspfeils mit der Bezugsziffer 70 bezeichnet. Das Symbol 70 wird jedoch nicht unmittelbar auf der Anzeigeeinheit 12 angezeigt. Vielmehr sendet der PC 16 die von ihm detektierten Mauskoordinaten an die Schnittstelleneinheit 66, wo sie mit Hilfe des Schnittstellenmoduls 68 in das Datenformat der Bildverarbeitungseinheit 52 umgesetzt werden. Die Bildverarbeitungseinheit 52 erhält ferner über den Ausgang 46 der Videokreuzschiene 32 das primäre Bild und überlagert diesem das Symbol 70. Das kombinierte Bild überträgt die Bildverarbeitungseinheit 52 anschließend als sekundäres Bild an die Videokreuzschiene 32 (Eingang 40). Von dort gelangt es über die Framegrabberkarte 18 wiederum zu der Anzeigeeinheit 12, auf der nun das mit dem Symbol 70 versehene sekundäre Bild 72 angezeigt wird. Der behandelnde Arzt kann dann weitere Markierungen oder Kommentare einfügen, die auf dieselbe Weise wieder auf der Anzeigeeinheit 12 angezeigt werden, wobei jeweils das anfänglich auf der Anzeigeeinheit 12 angezeigte Bild das primäre Bild im Sinne der vorliegenden Erfindung ist.

In dem hier gezeigten Ausführungsbeispiel wird das sekundäre Bild 72 außerdem auch auf der räumlich abgesetzten Anzeigeeinheit 54 angezeigt, die beispielsweise für Schulungszwecke oder auch für eine Datenarchivierung zur Verfügung steht.

Insgesamt ist zunächst eine Vorrichtung zum Erzeugen eines mit Bearbeitungsvermerken versehenen Bildes in einem sterilen Arbeitsbereich (56) einer medizinischen Einrichtung (10) offenbart, die Vorrichtung mit
- einer in dem sterilen Arbeitsbereich (56) angeordneten medizinischen Anzeigeeinheit (12) zum Darstellen eines primären Bildes,
- einem in dem sterilen Arbeitsbereich (56) angeordneten Eingabegerät (12) zum Erzeugen von Eingabedaten (24), wobei die Eingabedaten (24) den Bearbeitungsvermerken entsprechen, und
- einer außerhalb des sterilen Bereichs (56) angeordneten Bildverarbeitungseinheit (52), die dazu ausgebildet ist, die Eingabedaten (24) und das primäre Bild aufzunehmen und zu einem sekundären Bild (72) zusammenzuführen,
- wobei die medizinische Anzeigeeinheit (12) und die Bildverarbeitungseinheit (52) über eine Schnittstelle (18, 32) verbunden sind, so dass das sekundäre Bild (72) an die Anzeigeeinheit (12) übertragbar und dort darstellbar ist.

Ferner ist ein Schnittstellenmodul zur Verwendung in einer Vorrichtung nach Anspruch 9, das dazu ausgebildet ist, Eingabedaten (24) von einem in dem sterilen Arbeitsbereich (56) angeordneten Eingabegerät (12) in ein Datenformat für eine außerhalb des sterilen Arbeitsbereichs (56) angeordnete Bildverarbeitungseinheit (52) umzusetzen.

Schließlich ist ein Verfahren zum Erzeugen eines mit Bearbeitungsvermerken versehenen Bildes in einem sterilen Arbeitsbereich (56) einer medizinischen Einrichtung (10) offenbart, mit den Schritten:
- Darstellen eines primären Bildes auf einer medizinischen Anzeigeeinheit (12), die in dem sterilen Arbeitsbereich (56) angeordnet ist,
- Bereitstellen eines Eingabegerätes (12) zum Erzeugen von Eingabedaten (24) in dem sterilen Arbeitsbereich (56), wobei die Eingabedaten (24) den Bearbeitungsvermerken entsprechen,
- Einlesen der Eingabedaten (24) und Übertragen der Eingabedaten (24) an eine Bildverarbeitungseinheit (52) die außerhalb des sterilen Bereichs (56) angeordnet ist,
- Übertragen des primären Bildes an die Bildverarbeitungseinheit (52),
- Erzeugen eines sekundären Bildes (72) in der Bildverarbeitungseinheit (52) durch Zusammenführen des primären Bildes und der Eingabedaten (24), und
- Übertragen des sekundären Bildes (72) an die Anzeigeeinheit und Darstellen des sekundären Bildes (72) auf der Anzeigeeinheit (12).

Dabei ist es vorteilhaft, wenn das primäre Bild eine medizinische Aufnahme eines Patienten (28) ist, insbesondere eine in dem sterilen Arbeitsbereich (56) endoskopisch erzeugte Aufnahme.

Ferner ist es vorteilhaft, wenn als Eingabegerät ein berührungsempfindlicher Bildschirm (12) bereitgestellt wird, der vorzugsweise auch die Anzeigeeinheit bildet.

Ferner ist es vorteilhaft, wenn die Eingabedaten (24) über eine galvanische Trennstelle (30) an die Bildverarbeitungseinheit (52) übertragen werden.

Ferner ist es vorteilhaft, wenn die Eingabedaten (24) bei der Übertragung an die Bildverarbeitungseinheit (52) einer Protokollumsetzung unterzogen werden.

Ferner ist es vorteilhaft, wenn die Eingabedaten (24) in einem digitalen Format an die Bildverarbeitungseinheit (52) übertragen werden.

Ferner ist es vorteilhaft, wenn das primäre Bild als analoges Bildsignal an die Bildverarbeitungseinheit (52) übertragen wird.

Ferner ist es vorteilhaft, wenn das sekundäre Bild (72) auf einer zweiten Anzeigeeinheit (54) dargestellt wird, die außerhalb des sterilen Arbeitsbereichs (56) angeordnet ist.

## Patentansprüche

1. Vorrichtung zum Erzeugen eines mit Bearbeitungsvermerken versehenen Bildes in einem sterilen Arbeitsbereich (56) einer medizinischen Einrichtung (10), mit
- einer in dem sterilen Arbeitsbereich (56) angeordneten medizinischen Anzeigeeinheit (12) zum Darstellen eines primären Bildes,
- einem in dem sterilen Arbeitsbereich (56) angeordneten Eingabegerät (12) zum Erzeugen von Eingabedaten (24), wobei die Eingabedaten (24) den Bearbeitungsvermerken entsprechen, und
- einer außerhalb des sterilen Bereichs (56) angeordneten Bildverarbeitungseinheit (52), die dazu ausgebildet ist, die Eingabedaten (24) und das primäre Bild aufzunehmen und zu einem sekundären Bild (72) zusammenzuführen,
- wobei die medizinische Anzeigeeinheit (12) und die Bildverarbeitungseinheit (52) über eine Schnittstelle (18, 32) verbunden sind, so dass das sekundäre Bild (72) an die Anzeigeeinheit (12) übertragbar und dort darstellbar ist.

2. Vorrichtung nach Anspruch 1, **dadurch gekennzeichnet, dass** das primäre Bild eine medizinische Aufnahme eines Patienten (28) ist, insbesondere eine in dem sterilen Arbeitsbereich (56) endoskopisch erzeugte Aufnahme.

3. Vorrichtung nach Anspruch 1 oder 2, **dadurch gekennzeichnet, dass** als Eingabegerät ein berührungsempfindlicher Bildschirm (12) bereitgestellt wird, der vorzugsweise auch die Anzeigeeinheit bildet.

4. Vorrichtung nach einem der vorhergehenden Ansprüche, **gekennzeichnet durch** eine galvanische Trennstelle, über die Eingabedaten (24) an die Bildverarbeitungseinheit (52) übertragen werden.

5. Vorrichtung nach einem der vorhergehenden Ansprüche, **gekennzeichnet durch** galvanische Trennstellen, insbesondere Trennverstärker (30), die dafür ausgebildet sind, eine galvanische Trennung zwischen dem sterilen Arbeitsbereich (56) und einem nicht-sterilen Bereich (58) zu gewährleisten.

6. Vorrichtung nach Anspruch 5, **dadurch gekennzeichnet, dass** die galvanische Trennstellen an einer Trennung zwischen dem sterilen Arbeitsbereich (56) und dem nicht-sterilen Bereich (58) angeordnet sind.

7. Vorrichtung nach einem der vorhergehenden Ansprüche, **gekennzeichnet durch** einen Protokollumsetzer, der dafür ausgebildet ist, die Eingabedaten (24) bei der Übertragung an die Bildverarbeitungseinheit (52) einer Protokollumsetzung zu unterziehen.

8. Vorrichtung nach einem der vorhergehenden Ansprüche, **dadurch gekennzeichnet, dass** die Übertragung der Eingabedaten (24) an die Bildverarbeitungseinheit (52) in einem digitalen Format ausgebildet ist.

9. Vorrichtung nach einem der vorhergehenden Ansprüche, **dadurch gekennzeichnet, dass** die Übertragung des primären Bilds an die Bildverarbeitungseinheit (52) als analoges Bildsignal ausgebildet ist.

10. Vorrichtung nach einem der vorhergehenden Ansprüche, **dadurch gekennzeichnet, dass** außerhalb des sterilen Arbeitsbereichs (56) eine zweite Anzeigeeinheit (54) angeordnet, auf der das sekundäre Bild (72) dargestellt ist.

11. Vorrichtung nach einem der vorhergehenden Ansprüche, **gekennzeichnet durch** eine Kameraeinheit (26), die im sterilen Arbeitsbereich (56) zur Erzeugung des primären Bilds angeordnet ist.

12. Vorrichtung nach einem der Ansprüche 1 bis 10, **gekennzeichnet durch** eine Videokreuzschiene (32), die außerhalb des sterilen Arbeitsbereichs (56), insbesondere in einem nicht-sterilen Bereich (58), angeordnet ist und eine Kameraeinheit (26), die im sterilen Arbeitsbereich (56) zur Erzeugung des primären Bilds angeordnet ist, wobei die Kameraeinheit (26) über einen Trennverstärker (30) mit der Videokreuzschiene (32) verbunden ist.

13. Steriler Arbeitsbereich (56) und Vorrichtung nach einem der vorhergehenden Ansprüche, wobei die Anzeigeeinheit (12) und das Eingabegerät (12) in dem sterilen Arbeitsbereich (56) und die Bildverarbeitungseinheit (52) außerhalb des sterilen Arbeitsbereichs (56) angeordnet ist.

14. Steriler Arbeitsbereich (56), nicht-steriler Bereich (58) und Vorrichtung nach einem der Ansprüche 1 bis 12, wobei die Anzeigeeinheit (12) und das Eingabegerät (12) im sterilen Arbeitsbereich (56) und die Bildverarbeitungseinheit (52) im nicht-sterilen Bereich (58) angeordnet ist.

15. Medizinische Einrichtung (10) mit einem sterilen Bereich (56), mit einem nicht-sterilen Bereich (58) und mit einer Vorrichtung nach einem der Ansprüche 1 bis 12, wobei die Anzeigeeinheit (12) und das Eingabegerät (12) im sterilen Arbeitsbereich (56) und die Bildverarbeitungseinheit (52) im nicht-sterilen Bereich (58) angeordnet ist.
